(19) Europäisches Patentamt
European Patent Office
Office européen des brevets

(11) EP 2 119 395 A1

(12) EUROPEAN PATENT APPLICATION

(43) Date of publication:
18.11.2009 Bulletin 2009/47

(51) Int Cl.:
A61B 5/107 (2006.01)

(21) Application number: 09160370.4

(22) Date of filing: 15.05.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR

(30) Priority: 16.05.2008 US 122173

(71) Applicant: Jockey International, Inc.
Kenosha WI 53140 (US)

(72) Inventor: Chapman, Stewart
Coventry, West Midlands CV7 8RF (GB)

(74) Representative: Grünecker, Kinkeldey,
Stockmair & Schwanhäusser
Anwaltssozietät
Leopoldstrasse 4
80802 München (DE)

(54) **Breast volume measurement device and system**

(57) A measuring device (10) for measuring the volume of a breast (42) of a woman. The measuring device includes a plurality of arc-shaped elements (26) coupled together to allow each of the plurality of arc-shaped elements to pivot to different angular positions relative to the other of the plurality of arc-shaped elements. The plurality of arc-shaped elements are pivotable relative to each other between a closed position where the plurality of arc-shaped elements are generally aligned at a common angular position, and an open position where the plurality of arc-shaped elements are generally spaced from each other at different angular positions to at least partially define a portion of a spheroid having a predetermined volume. The plurality of arc-shaped elements in the open position are configured to be positioned against the breast to receive the breast for determining a volume of the breast relative to the predetermined volume.

FIG. 7

**Description**

BACKGROUND OF THE INVENTION

The present invention relates to measuring devices, and more particularly, to a system and method of measuring the volume of a breast of a woman.

SUMMARY OF THE INVENTION

Proper breast measurement, especially in terms of breast volume measurement can be advantageous. To measure the volume of the breast of a woman, a breast measuring device is used. The breast measuring device can provide a fast, effective and unobtrusive way to measure the female breast. Breast volume measurement can be used to properly fit a brassiere when cups of the brassiere are selected based on breast volume. Furthermore, a breast measuring device can be used for other purposes, including as part of breast reconstruction preparation and other design where reconstruction of the female physique is required.

In one embodiment, the invention provides a measuring device for measuring the volume of a breast of a woman. The measuring device includes a plurality of arc-shaped elements coupled together to allow each of the plurality of arc-shaped elements to pivot to different angular positions relative to the other of the plurality of arc-shaped elements. The plurality of arc-shaped elements are pivotable relative to each other between a closed position where the plurality of arc-shaped elements are generally aligned at a common angular position, and an open position where the plurality of arc-shaped elements are generally spaced from each other at different angular positions to at least partially define a portion of a spheroid having a predetermined volume. The plurality of arc-shaped elements in the open position are configured to be positioned against the breast to receive the breast for determining a volume of the breast relative to the predetermined volume.

In another embodiment, the invention provides a measuring system for measuring the volume of a breast of a woman. The measuring system includes a plurality of breast measuring devices. Each of the plurality of breast measuring devices includes a plurality of arc-shaped elements coupled together to allow each of the plurality of arc-shaped elements to pivot to different angular positions relative to the other of the plurality of arc-shaped elements. The plurality of arc-shaped elements are pivotable relative to each other between a closed position where the plurality of arc-shaped elements are generally aligned at a common angular position, and an open position where the plurality of arc-shaped elements are generally spaced from each other at different angular positions to at least partially define a portion of a spheroid having a predetermined volume. The plurality of arc-shaped elements in the open position are configured to be positioned against the breast to receive the breast for determining a volume of the breast relative to the predetermined volume.

In another embodiment, the invention provides a method of measuring the volume of a breast of a woman. The method includes pivoting a plurality of arc-shaped elements of a first breast measuring device to an open position where the plurality of arc-shaped elements are generally spaced from each other at different angular positions, defining a portion of a spheroid having a first predetermined volume when the plurality of arc-shaped elements of the first breast measuring device are in the open position, positioning the first breast measuring device against the breast of the woman when the plurality of arc-shaped elements of the first breast measuring device are in the open position, and comparing the fit of the first breast measuring device on the breast to determine the breast volume.

Other aspects of the invention will become apparent by consideration of the detailed description and accompanying drawings.

BRIEF DESCRIPTION OF THE DRAWINGS

Fig. 1 illustrates a side perspective view of a breast measuring device of the present invention in an open position.
Fig. 2 is a side view of the breast measuring device of Fig. 1.
Fig. 2A is a side view of a female breast.
Fig. 3 is a side perspective view of a breast measuring device of the present invention in a closed position.
Fig. 3A is a cross-sectional view of the breast measuring device of Fig. 3.
Fig. 4 is a top perspective view of a breast measuring system of the present invention.
Fig. 5 is a side view of the breast measuring system of Fig. 4.
Fig. 6 is a top perspective view of the breast measuring system of Fig. 4 with one of the breast measuring devices removed from a holder and in the open position.
Fig. 7 is a front view of a breast measuring device of the present invention as shown on a female user in an open position.
Fig. 8 is a side perspective view of another embodiment of the breast measuring device of the present invention in an open position.

Fig. 9 illustrates a side perspective view of an alternate embodiment of a breast measuring device of the present invention in an open position.

Fig. 10 is a side view of the breast measuring device of Fig. 9.

Fig. 11 is a front view of the breast measuring device of Fig. 9.

Fig. 12 is a side perspective view of the breast measuring device of Fig. 9 in a closed position.

Fig. 13 is a side view of the breast measuring device of Fig. 12.

Fig. 14 is a front view of the breast measuring device of Fig. 12.

DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting.

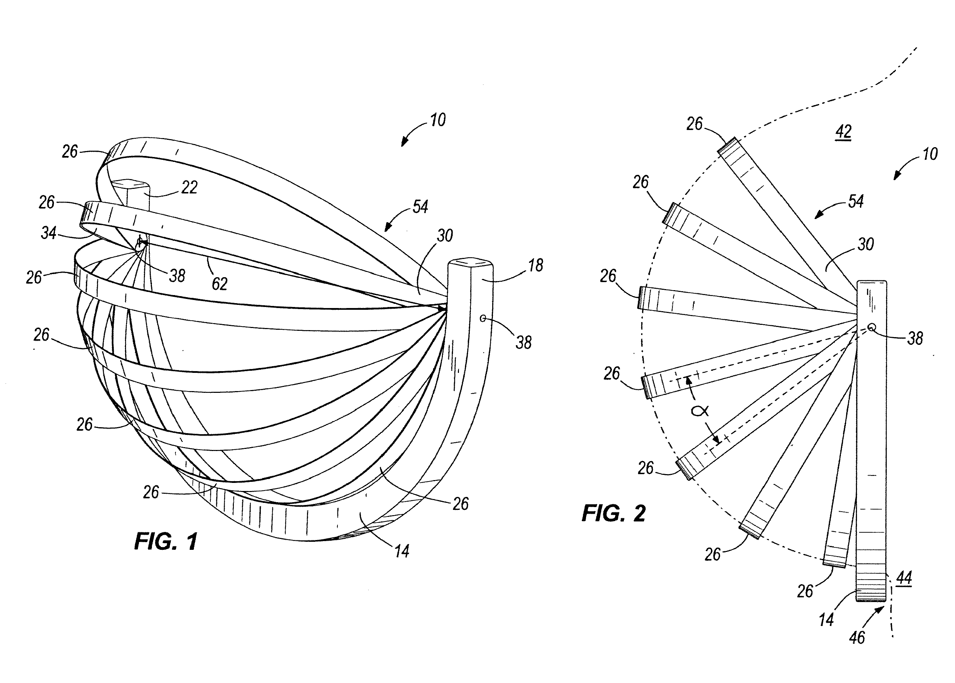

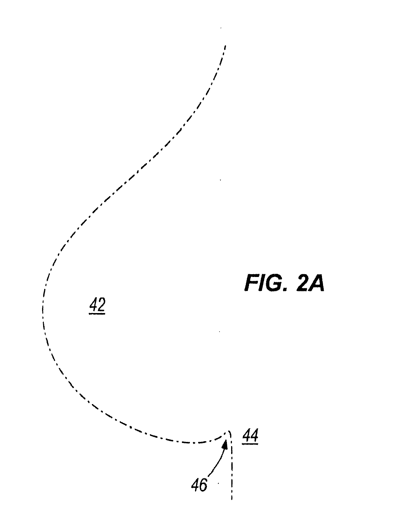

Fig. 1 illustrates one embodiment of the breast measuring device 10 of the present invention. The breast measuring device 10 is configured to measure the breast 42 of a female. The breast measuring device 10 includes an arc-shaped support 14 and a plurality of arc-shaped elements 26 coupled together with fasteners 38. The arc-shaped support 14 has a first end 18 and a second end 22 and is configured to be positioned against a chest area 44 of the female. The arc-shaped support 14 is also positioned against an inframammary fold 46 of the female. As shown in Fig. 2A, the inframammary fold 46 is the crease under the breast 42 where the breast 42 transitions to the chest area 44. The arc-shaped support 14 is rigid and formed of plastic. However, in other embodiments, the arc-shaped support can be formed of metal or other material. Each of the plurality of arc-shaped elements 26 has a first end 30 and a second end 34.

As shown in Fig. 2, the plurality of arc-shaped elements 26 are coupled together to allow each of the plurality of arc-shaped elements 26 to pivot to different angular positions relative to the other of the plurality of arc-shaped elements 26. The different angular positions of the plurality of arc-shaped elements 26 defines angles α between adjacent pairs of the plurality of arc-shaped elements 26. Each of the plurality of arc-shaped elements 26 is individually movable and positionable. Furthermore, each of the plurality of arc-shaped elements 26 is flexible and formed of plastic. However, in other embodiments, each of the plurality of arc-shaped elements can be formed of metal, cloth, or other material. The fastener 38 is shown as a pin, although any suitable fastener can be used. The fastener 38 is configured to couple the first end 30 and second end 34 of each of the plurality of arc-shaped elements 26 at the first end 18 and second end 22, respectively, of the arc-shaped support 14. The fastener 38 is further configured to permit movement and positioning of the arc-shaped elements.

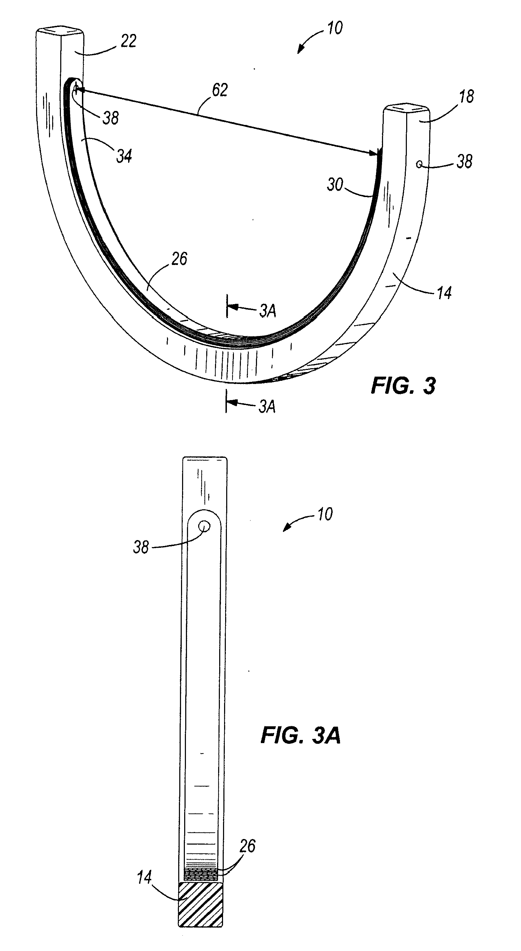

As shown in Figs. 1-3 and 3A, the plurality of arc-shaped elements 26 are pivotable relative to each other between a closed position where the plurality of arc-shaped elements 26 are generally aligned at a common angular position (see Fig. 3) and an open position where the plurality of arc-shaped elements 26 are generally spaced from each other at different angular positions to at least partially define a portion of a spheroid 54, or a spherical wedge, having a predetermined volume (see Fig. 2). The breast measuring device 10 can have any number of arc-shaped elements 26. The plurality of arc-shaped elements 26 can also be positioned at any of a plurality of positions between the fully closed position and the fully open position, such as by way of example only, in a semi-open position. As shown in Fig. 3A, the arc-shaped elements are configured to generally align at a common angular position, such that each of the plurality of arc-shaped elements are nested within each other and are touching each other.

In other constructions, the thickness of the arc-shaped elements may not allow for this type of nesting because portions of adjacent arc-shaped elements may interfere with each other when moved toward a common angular position. In this case, the closed position shall mean a position where the arc-shaped elements are moved toward a common angular position until adjacent arc-shaped elements contact each other prior to nesting. In this closed position, the arc-shaped elements shall be considered to be generally aligned at a common angular position even though the angular positions of the arc shaped elements are slightly different.

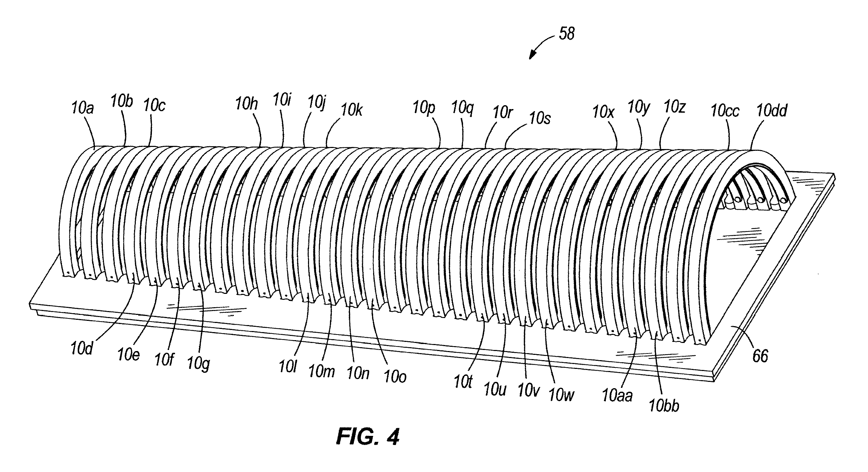

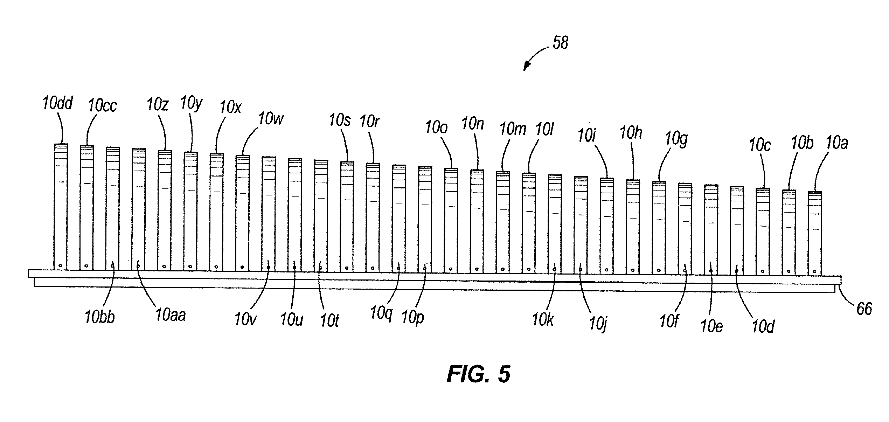

Figs. 4-6 show the breast measuring device 10 as part of a system or set 58 of breast measuring devices. Each of the breast measuring devices 10 includes the arc-shaped support 14, the plurality of arc-shaped elements 26 and fasteners 38 as described above with respect to Figs. 1-3 and 3A. Each of the breast measuring devices 10 corresponds to a predetermined breast volume that is different from the predetermined breast volume of the other breast measuring devices in the system. Each of the breast measuring devices in the system 58 has a specific diameter 62 ranging from the smallest device of 100 millimeters to the largest device of 158 millimeters. The diameters 62 of the devices 10 in the system are sized in two millimeter increments (10a-10dd, respectively). Additional breast measuring devices can be added to the system that are of smaller diameter than 100 mm or a larger diameter than 158 millimeters. In other embodiments, the breast measuring devices may range from greater or less than 2 millimeter increment sizing between the breast measuring devices within the system.

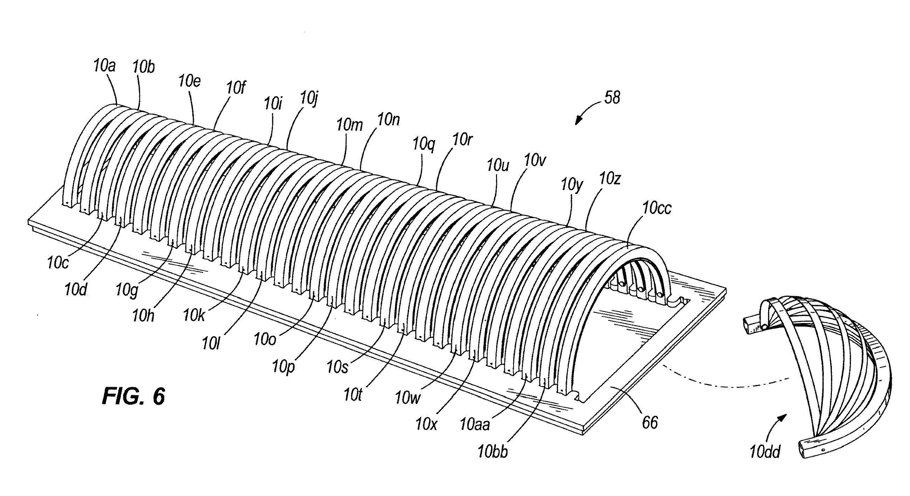

As shown in Figs. 4-6, the breast measuring devices 10 of the system 58 are adapted to fit within a holder 66 such that each device 10 stands vertically. The holder 66 is configured to hold the system 58 of breast measuring devices 10 in an accessible manner that makes each of the breast measuring devices 10 easy to remove and replace by a user. Fig. 6 shows one of the breast measuring devices 10dd of the system of breast measuring devices removed from the holder 66 and in an open position.

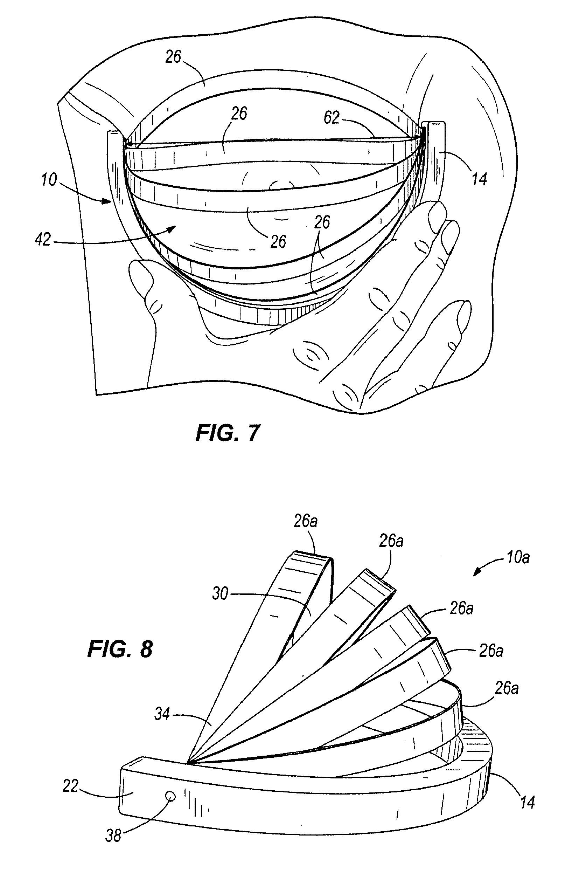

Each of the breast measuring devices in the system of breast measuring devices 58 can have any of a plurality of arc-shaped elements 26. In the illustrated embodiments, the smaller diameter breast measuring devices have less arc-shaped elements than the larger diameter breast measuring devices. Similarly, in the illustrated embodiments, a smaller diameter breast measuring device 10a (see Fig. 8) may have a wider arc-shaped element 26a than a larger diameter breast measuring device 10 (see Fig. 1). However, each breast measuring device can have any number of arc-shaped elements and the width of arc-shaped element can be consistent with the other arc-shaped elements 26 of the device 10 or may be different from the other arc-shaped elements 26 of the device 10. For example, the widths of the arc-shaped elements 26 of a single device 10 may vary from narrowest to widest in the direction away from the arc-shaped support 14.

Each of the breast measuring devices 10 corresponds to a pre-determined breast volume. The breast measuring device volume is determined by calculating the volume of a half sphere. The device volume is determined by the following calculations:

Volume calculation for a whole sphere:

$$V = (D^3 * pi) / 6$$

Volume calculation for half-sphere for a known device diameter:

$$V = [(D^3 * pi) / 2] / 6$$

If D = 10.0 millimeter, then the volume equals:

$$V = [(10.0^3 * 3.14159) / 2] / 6 = 261.83 \ cc$$

Table 1 illustrates the breast measuring device diameter and corresponding volume measurements for the breast measuring device system 58 as calculated using the above formulas.

Table 1

| Diameter (mm) | Volume (cc) |
| --- | --- |
| 100 | 261.83 |
| 102 | 277.86 |
| 104 | 294.53 |
| 106 | 311.85 |
| 108 | 329.83 |
| 110 | 348.50 |
| 112 | 367.86 |
| 114 | 387.92 |
| 116 | 408.69 |
| 118 | 430.20 |
| 120 | 452.45 |

(continued)

| Diameter (mm) | Volume (cc) |
| --- | --- |
| 122 | 475.45 |
| 124 | 499.22 |
| 126 | 523.77 |
| 128 | 549.10 |
| 130 | 575.25 |
| 132 | 602.21 |
| 134 | 630.00 |
| 136 | 658.63 |
| 138 | 688.12 |
| 140 | 718.47 |
| 142 | 749.70 |
| 144 | 781.83 |
| 146 | 814.86 |
| 148 | 848.81 |
| 150 | 883.69 |
| 152 | 919.51 |
| 154 | 956.28 |
| 156 | 994.03 |
| 158 | 1032.75 |

In operation and as shown in Fig. 7, the breast measuring device 10 is used to determine the volume measurement of a breast 42. To determine the volume of the breast 42 of a woman, one of the plurality of breast measuring devices 10 is chosen from the system of breast measuring devices 58 and the arc-shaped elements 26 are pivoted from the closed position to the open position. In some embodiments, the arc-shaped elements are opened to substantially defining a ½ sphere such as illustrated in Figs. 1 and 2. In other embodiments, the arc-shaped elements are opened to define less than ½ sphere, such as ¼ sphere. The breast measuring device 10 is held against the chest area 50 such that the arc-shaped support 14 is positioned against the inframammary fold 46 of the breast 42. As shown in Fig. 7, the breast measuring device 10 is used with a nude breast.

Once positioned against the inframammary fold 46 of the breast 42, the volume of the breast 42 can be compared to the size of the device 10. The arc-shaped elements 26 are flexible to enable positioning about the circumference of the breast 42. The breast 42 should fit within the breast measuring device 10 when the breast measuring device 10 is in the open position such that the breast 42 does not protrude through the arc-shaped elements 26, which would indicate that the volume of the breast is larger than the volume of the breast measuring device 10. Alternatively, excess space between the breast 42 and the breast measuring device 10 would indicate that the volume of the breast is less than the volume of the breast measuring device. In such circumstances, the operator shall pick a different breast measuring device with a different volume from the system of breast measuring devices 58. Measuring of the breast volume is continued in this manner until the correct breast volume is determined. When the breast measuring device is properly fitted to the breast, the open position breast measuring device will resemble the natural breast in terms of, by way of example only, breast volume and breast contour.

In other applications, the breast measuring system 58 may be used to determine the proper fitting brassiere using a breast volume measurement system. For example, the system of breast measuring devices can correspond to the grading array groups described in U.S. Patent Application Serial No. 12/035,833 filed on February 22, 2008 and entitled "SYSTEM AND METHOD OF CONSTRUCTING AND SIZING BRASSIERES," the entire contents of which are hereby incorporated by reference. More specifically, Table 2 shows the breast measuring device diameters which correspond to the volume groups in the grading array group.

Table 2

| Device Diameter (mm) | Volume Group Number | Volume (cc) |
|---|---|---|
| 92 | 1 | 203 |
| 110 | 2 | 348 |
| 124 | 3 | 499 |
| 136 | 4 | 658 |
| 146 | 5 | 814 |
| 156 | 6 | 994 |
| 164 | 7 | 1154 |
| 172 | 8 | 1332 |

The breast measuring devices may also be used to assist in illustrating a desired breast volume. For example, in the event of breast augmentation, the patient and doctor may want to model what the reconstructed breast will resemble. For example, the breast of the patient can be measured using the system of breast measuring devices 58 to obtain and assign a volume to the unaugmented breast. The patient will then select a doctor proposed or patient desired breast volume. A brassiere can be provided to the patient having the proposed breast volume cup size, which may or may not include the proper band size measurement for the patient or may include an adjustable band. The patient will then try on the brassiere and insert padding as needed to fill the volume of the brassiere cup. The patient can then try on clothing over the brassiere to see how the chest area will likely look with the proposed breast volume size. This process can be repeated until the desired appearance and breast volume are obtained. In this manner, a patient or doctor can have a more accurate idea of the eventual outcome of the breast augmentation.

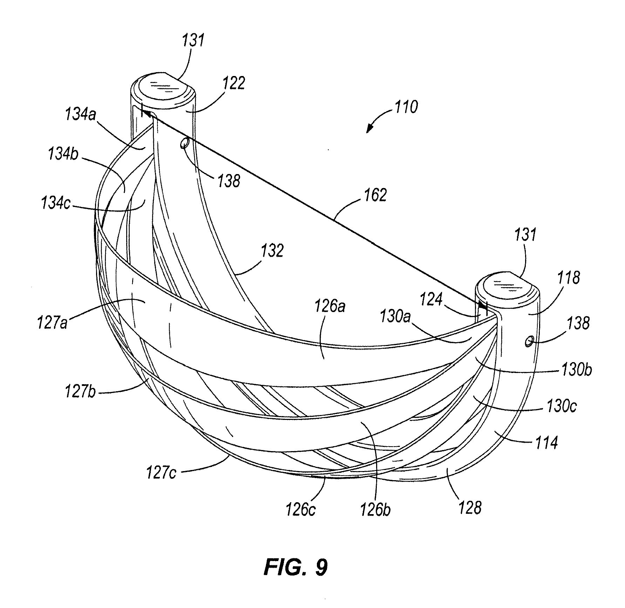

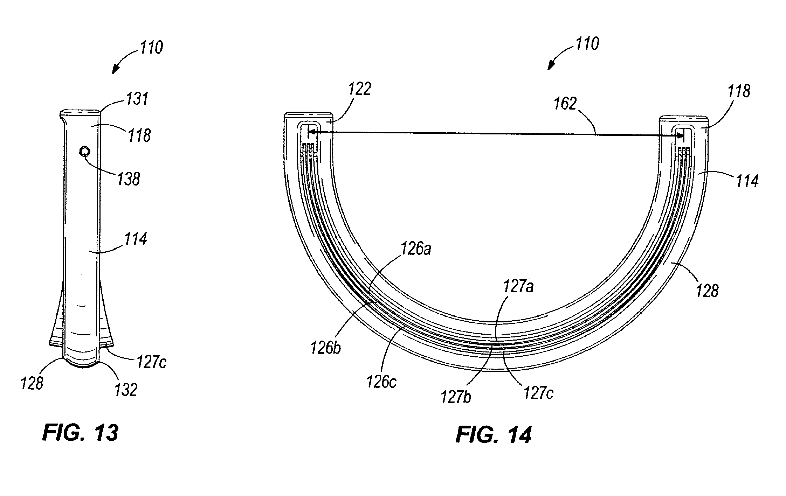

Figs. 9-14 illustrate another embodiment of a breast measuring device 110 according to the invention. The breast measuring device 110 shown in Figs. 9-14 includes similar structure to the breast measuring device 10 illustrated in Figs. 1-8 described above; therefore, like structure is identified by the same reference numeral. Accordingly, with the exception of mutually inconsistent features and elements between the embodiment of Figs. 9-14 and the embodiments of Figs. 1-8, reference is hereby made to the description above accompanying the embodiments of Figs. 1-8 for a more complete description of the features and elements (and the alternatives to the features and elements) of the embodiment of Figs. 9-14. Furthermore, the breast measuring device 110 is configured as part of a system or set of breast measuring devices similar to the system 58 described above with respect to breast measuring device 10.

The breast measuring device 110 includes an arc-shaped support 114 and a plurality of arc-shaped elements 126a, 126b, and 126c coupled together with fasteners 138. The arc-shaped support 114 has a first end 118, a second end 122, and a slot 124 extending through the arc-shaped support 114 from a front end 128 to a back end 132 of the arc-shaped support 114. The back end 132 of the arc-shaped support 114 has a flat surface 131 configured to be positioned against the chest area 44 of the woman and adjacent the inframammary fold 46. Each of the arc-shaped elements 126a, 126b, and 126c has a first end 130a, 130b, and 130c, respectively, and a second end 134a, 134b, and 134c, respectively. The arc-shaped support 114 is shown with three arc-shaped elements 126a, 126b, and 126c. However, in other embodiments, the arc-shaped support 114 may have more or less than three arc-shaped elements 126a, 126b, and 126c.

Each of the breast measuring devices in the system has a specific diameter 162 ranging from the smallest device of 100 millimeters to the largest device of 158 millimeters. The diameters 162 of the devices 100 in the system are sized in two millimeter increments. Additional breast measuring devices can be added to the system that are of smaller diameter than 100 mm or a larger diameter than 158 millimeters. In other embodiments, the breast measuring devices may range from greater or less than 2 millimeter increment sizing between the breast measuring devices within the system.

The plurality of arc-shaped elements 126a, 126b, and 126c are coupled together to allow each of the plurality of arc-shaped elements 126a, 126b, and 126c to pivot to different angular positions relative to the other of the plurality of arc-shaped elements 126a, 126b, and 126c. The different angular positions of the plurality of arc-shaped elements 126a, 126b, and 126c define angle $\alpha$ between adjacent pairs of the plurality of arc-shaped elements 126a, 126b, and 126c. Each of the plurality of arc-shaped elements 126a, 126b, and 126c is individually movable and positionable. Each of the plurality of arc-shaped elements 126a, 126b, and 126c is further configured to nest within the adjacent arc-shaped element 126a, 126b, 126c when positioned within slot 124 of the arc-shaped support 114. For instance, when positioned within slot 124 of the arc-shaped support 114, arc-shaped element 126a is substantially adjacent and nesting within arc-shaped element 126b and arc-shaped element 126b is substantially adjacent and nesting within arc-shaped element 126c.

Figs. 9-11 illustrate that each of the arc-shaped elements 126a, 126b, and 126c has a center portion 127a, 127b, and 127c that is wider than each of the first end 130a, 130b, and 130c, respectively, and a second end 134a, 134b, and 134c, respectively. Additionally, each of the arc-shaped elements 126a, 126b, and 126c has a different center portion 127a, 127b, and 127c, such that for example, center portion 127c is wider than both of center portions 127b, 127a, and center portion 127b is wider than center portion 127a. However, in other embodiments, each of the arc-shaped elements 126a, 126b, and 126c may have the same center portion 127a, 127b, and 127c.

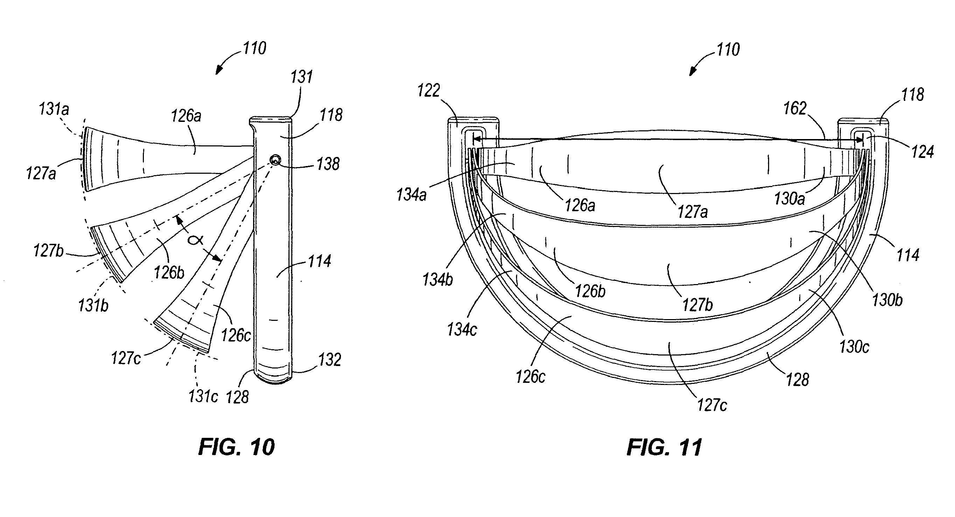

As shown in Fig. 10, each of the arc-shaped elements 126a, 126b, and 126c has a curvature 131 a, 131 b, 131 c, respectively, such that the center portions 127a, 127b, and 127c are slightly curved or arc-shaped. The center portions 127a, 127b, and 127c are slightly curved to enable a more accurate measurement of the breast volume because the curvature 131 a, 131b, 131 c of each of the center portions 127a, 127b, and 127c allows the arc-shaped element 126a, 126b, and 126c to more closely conform to the curvature of the breast. In other embodiments, the arc-shaped elements may be substantially flat without the curvature. The curvature 131a, 131b, 131c may be the same for each of the arc-shaped elements 126a, 126b, and 126c of the breast measuring device 110 or the curvature 131 a, 131 b, 131c may be different for each of the arc-shaped elements 126a, 126b, and 126c of the breast measuring device 110.

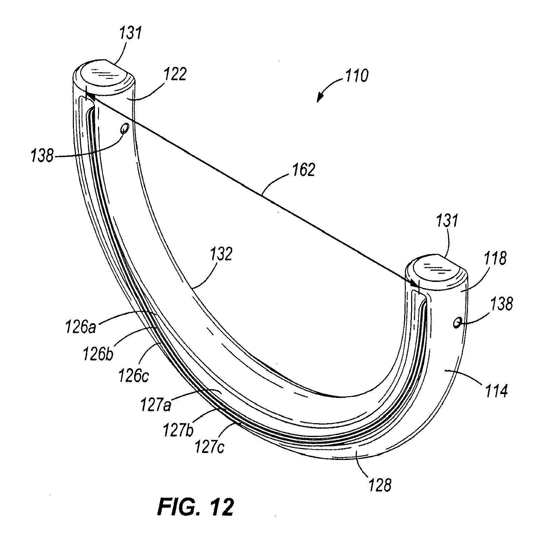

Figs. 12-14 show that the center portions 127a, 127b, and 127b of each of the plurality of arc-shaped elements 126a, 126b, and 126c are wider than the width of the arc-shaped support 114, such that the center portion 127a, 127b, 127c of each arc-shaped element 126a, 126b, and 126c extends from both the front end 128 and the back end 132 of the arc-shaped support 114 when the arc-shaped elements 126a, 126b, and 126c are positioned within the slot 124. In the illustrated embodiments, the center portions 127a, 127b, and 127c are wider than the arc-shaped support. The arc-shaped elements 126a, 126b, and 126c are generally wider than the arc-shaped support 114 to allow the user to grasp the center portions 127a, 127b, and 127c to manipulate the arc-shaped elements 126a, 126b, and 126c when the arc-shaped elements 126a, 126b, and 126c are positioned within the slot 124.

Various features and advantages of the invention are set forth in the following claims.

**Claims**

1. A measuring device for measuring the volume of a breast of a woman, the measuring device comprising:

    a plurality of arc-shaped elements coupled together to allow each of the plurality of arc-shaped elements to pivot to different angular positions relative to the other of the plurality of arc-shaped elements, the plurality of arc-shaped elements pivotable relative to each other between a closed position where the plurality of arc-shaped elements are generally aligned at a common angular position, and an open position where the plurality of arc-shaped elements are generally spaced from each other at different angular positions to at least partially define a portion of a spheroid having a predetermined volume, the plurality of arc-shaped elements in the open position configured to be positioned against the breast to receive the breast for determining a volume of the breast relative to the predetermined volume.

2. The measuring device of claim 1, further comprising an arc-shaped support coupled to the plurality of arc-shaped elements to allow each of the plurality of arc-shaped elements to pivot relative to the arc-shaped support.

3. The measuring device of claim 2, wherein the arc-shaped support is rigid.

4. The measuring device of claim 1, wherein each of the plurality of arc-shaped elements is flexible,

5. The measuring device of claim 2, wherein the arc-shaped support is configured to be positioned against an inframammary fold of the woman when the plurality of arc-shaped elements are in the open position and positioned against the breast.

6. The measuring device of claim 1, wherein each of the plurality of arc-shaped elements includes a first end and a second end pivotally connected to the arc-shaped support with fasteners.

7. The measuring device of claim 1, wherein the portion of the spheroid is a spherical wedge.

8. A measuring system for measuring the volume of a breast of a woman, the measuring device system comprising:

a plurality of breast measuring devices, each of the plurality of breast measuring devices including a plurality of arc-shaped elements coupled together to allow each of the plurality of arc-shaped elements to pivot to different angular positions relative to the other of the plurality of arc-shaped elements, the plurality of arc-shaped elements pivotable relative to each other between a closed position where the plurality of arc-shaped elements are generally aligned at a common angular position, and an open position where the plurality of arc-shaped elements are generally spaced from each other at different angular positions to at least partially define a portion of a spheroid having a predetermined volume, the plurality of arc-shaped elements in the open position configured to be positioned against the breast to receive the breast for determining a volume of the breast relative to the predetermined volume.

9. The measuring system of claim 8, wherein each of the plurality of breast measuring devices further comprises an arc-shaped support coupled to the plurality of arc-shaped elements to allow each of the plurality of arc-shaped elements to pivot relative to the arc-shaped support.

10. The measuring system of claim 9, wherein the arc-shaped support of each of the plurality of breast measuring devices is configured to be positioned against an inframammary fold of the woman when the plurality of arc-shaped elements are in the open position and positioned against the breast.

11. The measuring system of claim 9, wherein each of the plurality of arc-shaped elements of each of the plurality of breast measuring devices includes a first end and a second end pivotally connected to the arc-shaped support with fasteners.

12. The measuring system of claim 8, wherein each of the plurality of breast measuring devices defines a predetermined volume measurement that is different from the predetermined volume measurement of the other breast measuring devices.

13. The measuring system of claim 8, wherein each of the plurality of breast measuring devices has a diameter different from the diameter of the other of the plurality of breast measuring devices.

14. The measuring system of claim 13, wherein each of the plurality of breast measuring devices has a diameter that is at least two millimeters different than another of the plurality of breast measuring devices.

15. The measuring system of claim 9, wherein each arc-shaped support of each of the plurality of breast measuring devices is rigid.

16. The measuring of claim 8, wherein each of the plurality of arc-shaped elements of each of the plurality of breast measuring devices is flexible.

17. A method of measuring the volume of a breast of a woman, the method comprising:

pivoting a plurality of arc-shaped elements of a first breast measuring device to an open position where the plurality of arc-shaped elements are generally spaced from each other at different angular positions;
defining a portion of a spheroid having a first predetermined volume when the plurality of are-shaped elements of the first breast measuring device are in the open position;
positioning the first breast measuring device against the breast of the woman when the plurality of arc-shaped elements of the first breast measuring device are in the open position; and
comparing the fit of the first breast measuring device on the breast to determine the breast volume.

18. The method of claim 17, further comprising:

determining that the breast of the woman is smaller than the first predetermined volume;
pivoting a plurality of arc-shaped elements of a second breast measuring device to an open position where the plurality of arc-shaped elements of the second breast measuring device are generally spaced from each other at different angular positions;
defining a portion of a spheroid having a second predetermined volume smaller than the first predetermined volume when the plurality of arc-shaped elements of the second breast measuring device are in the open position;
positioning the second breast measuring device against the breast of the woman when the plurality of arc-shaped elements of the second breast measuring device are in the open position; and

comparing the fit of the second breast measuring device on the breast to determine the breast volume.

19. The method of claim 17, further comprising:

determining that the breast of the woman is larger than the first predetermined volume;
pivoting a plurality of arc-shaped elements of a second breast measuring device to an open position where the plurality of arc-shaped elements of the second breast measuring device are generally spaced from each other at different angular positions;
defining a portion of a spheroid having a second predetermined volume larger than the first predetermined volume when the plurality of arc-shaped elements of the second breast measuring device are in the open position;
positioning the second breast measuring device against the breast of the woman when the plurality of arc-shaped elements of the second breast measuring device are in the open position; and
comparing the fit of the second breast measuring device on the breast to determine the breast volume.

20. The method of claim 19, further comprising:

determining that the breast of the woman is smaller than the second predetermined volume;
pivoting a plurality of arc-shaped elements of a third breast measuring device to an open position where the plurality of arc-shaped elements of the third breast measuring device are generally spaced from each other at different angular positions;
defining a portion of a spheroid having a third predetermined volume smaller than the second predetermined volume and larger than the first predetermined volume when the plurality of arc-shaped elements of the third breast measuring device are in the open position;
positioning the third breast measuring device against the breast of the woman when the plurality of arc-shaped elements of the third breast measuring device are in the open position; and
comparing the fit of the third breast measuring device on the breast to determine the breast volume.

FIG. 2

FIG. 1

FIG. 2A

42

44

46

**FIG. 3**

**FIG. 3A**

FIG. 4

**FIG. 5**

EP 2 119 395 A1

*FIG. 6*

**FIG. 7**

**FIG. 8**

**FIG. 9**

**FIG. 10**

**FIG. 11**

EP 2 119 395 A1

**FIG. 12**

FIG. 14

FIG. 13

Europäisches
Patentamt

European
Patent Office

Office européen
des brevets

## EUROPEAN SEARCH REPORT

Application Number

EP 09 16 0370

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/215101 A1 (RIOUX ROBERT [US] ET AL) 28 October 2004 (2004-10-28) * paragraph [0103] - paragraph [0106]; figure 22 * | 1-3, 5-11,15 | INV. A61B5/107 |
| A | US 5 485 855 A (SHIRAIWA NORINOBU [JP] ET AL) 23 January 1996 (1996-01-23) * figures * | 1,8,17 | |
| A | US D 546 719 S1 (LALONDE DONALD H [CA]) 17 July 2007 (2007-07-17) * figures * | 1,8,17 | |
| A | US 867 011 A (WILLIAM S. BROMLEY) 24 September 1907 (1907-09-24) * figures * | 1,8,17 | |
| A | US 2004/049435 A1 (NABARRO DANIEL JOSEPH NUNES [GB]) 11 March 2004 (2004-03-11) * figures * | 1,8,17 | |

TECHNICAL FIELDS
SEARCHED      (IPC)

A61B
A41H
G01B

The present search report has been drawn up for all claims

| Place of search | Date of completion of the search | Examiner |
|---|---|---|
| Munich | 28 July 2009 | Strubel, Christine |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

EP 2 119 395 A1

## ANNEX TO THE EUROPEAN SEARCH REPORT
## ON EUROPEAN PATENT APPLICATION NO.

EP 09 16 0370

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2009

| Patent document cited in search report | | Publication date | Patent family member(s) | | Publication date |
|---|---|---|---|---|---|
| US 2004215101 | A1 | 28-10-2004 | EP | 1615579 A2 | 18-01-2006 |
| | | | WO | 2004093705 A2 | 04-11-2004 |
| US 5485855 | A | 23-01-1996 | JP | 2561896 Y2 | 04-02-1998 |
| | | | JP | 6069227 U | 27-09-1994 |
| US D546719 | S1 | 17-07-2007 | NONE | | |
| US 867011 | A | | NONE | | |
| US 2004049435 | A1 | 11-03-2004 | NONE | | |

EPO FORM P0459

For more details about this annex : see Official Journal of the European Patent Office, No. 12/82

22

**EP 2 119 395 A1**

**REFERENCES CITED IN THE DESCRIPTION**

**Patent documents cited in the description**

- US 03583308 A